# EUROPEAN PATENT APPLICATION

(11) **EP 2 832 854 A1**
(43) Date of publication of application: **04.02.2015**
(21) Application number: 13179034.7
(22) Date of filing: 02.08.2013
(51) Int. Cl.: C12N 9/64, C12N 15/67, C12N 15/85

(54) **Method for improving the recombinant expression of a polypeptide by C-terminal fusion to human neprilysin**

(71) Applicant: F. Hoffmann-La Roche AG, 4070 Basel (CH)
(72) Inventor: Knoetgen, Hendrik, 82377 Penzberg (DE); Niewoehner, Jens, 81476 München (DE)
(74) Representative: Burger, Alexander

(57) **Abstract**

Herein is reported a method for the recombinant production of a polypeptide in a mammalian cell comprising the following steps i) cultivating a mammalian cell expressing the polypeptide as fusion polypeptide with human neprilysin whereby the neprilysin is fused to the C-terminus of the polypeptide, and ii) recovering the polypeptide from the cell or the cultivation medium and thereby producing the polypeptide.

## Description

The current invention is in the field of recombinant polypeptide production. Herein is reported a method for the recombinant production of a polypeptide with increased yield by expressing the polypeptide as C-terminal fusion polypeptide with human neprilysin or a neprilysin fragment.

### Background of the Invention

In recent years the production of therapeutic polypeptides has steadily increased and it is likely that therapeutic polypeptides will become the biggest group of therapeutics available for the treatment of various diseases in the near future. The impact of therapeutic polypeptides emerges from their specificity, such as specific target recognition and/or binding function.

Cell cultures are used in fermentative processes to produce substances and in particular polypeptides. A distinction is made between processes in which the cell cultures are genetically unmodified and form their own metabolic products and processes in which the organisms are genetically modified in such a manner that they either produce a larger amount of their own substances such as polypeptides or produce foreign substances. The organisms producing the substances are supplied with a nutrient medium which guarantees the survival of the organisms and enables the production of the desired target compound.

### Summary of the Invention

It has been found that by fusing human neprilysin or a fragment thereof to the C-terminus of a polypeptide the recombinant yield of the polypeptide can be increased when expressed using a mammalian cell.

One aspect as reported herein is a method for the recombinant production of a polypeptide in a mammalian cell comprising the following steps:
- cultivating a mammalian cell expressing the polypeptide as fusion polypeptide with human neprilysin whereby the neprilysin is fused to the C-terminus of the polypeptide, and
- recovering the polypeptide from the cell or the cultivation medium and thereby producing the polypeptide.

One aspect as reported herein is the use of human neprilysin fused to the C-terminus of a polypeptide for increasing the expression yield of the polypeptide in a mammalian cell.

In the following embodiments of all aspect as reported herein are given. It is pointed out that all embodiments can be combined with each other. This expressly includes all possible permutations of combinations of the given embodiments.

In one embodiment the neprilysin is a neprilysin fragment consisting of amino acid residues 52 to 750 of SEQ ID NO: 04.

In one embodiment the neprilysin is fused via a linker polypeptide to the C-terminus of the polypeptide. In one embodiment the linker is a glycine-serine-linker.

In one embodiment the fusion polypeptide comprises an enzymatic cleavage site between the polypeptide and the neprilysin or the linker.

In one embodiment the enzymatic cleavage site comprises a fragment of the cleavage site of Immunoglobulin A protease (IgA protease).

In one embodiment the mammalian cell is a HEK cell or a CHO cell.

In one embodiment the method comprises the following steps:
- cultivating a mammalian cell expressing the polypeptide as fusion polypeptide with human neprilysin whereby the neprilysin is fused to the C-terminus of the polypeptide,
- recovering the polypeptide from the cell or the cultivation medium, and
- enzymatically cleaving the fusion polypeptide and thereby producing the polypeptide.

In one embodiment the polypeptide is a biologically active polypeptide.

In one embodiment the polypeptide is an antibody. In one embodiment the neprilysin is fused to the C-terminus of one heavy chain of the antibody.

### FIGURES

- **Figure 1**: Binding of mAb31 and trifunctional antibody TriGant to Aβ1-40 fibers in vitro by ELISA; control antibody is a mouse transferrin receptor antibody (mouse TfR).
- **Figure 2**: Binding of mAb31 and trifunctional antibody TriGant to murine transferrin receptor in vitro by FACS analysis.
- **Figure 3**: Enzymatic activity of Neprilysin (R&D Systems, Cat. No 1182-ZNC) and Neprilysin TriGant antibody in vitro.
- **Figure 4**: Immunohistochemical staining of mAb31 and trifunctional antibody TriGant bound to native human β-amyloid plaques from brain sections of an Alzheimer's disease patient.
- **Figure 5**: In vivo β-amyloid plaque decoration by mAb31 and trifunctional antibody TriGant in a mouse model of Alzheimer's disease.

### Detailed Description of the Invention

Methods and techniques known to a person skilled in the art, which are useful for carrying out the current invention, are described e.g. in Ausubel, F.M., ed., Current Protocols in Molecular Biology, Volumes I to III (1997), Wiley and Sons; Sambrook et al., Molecular Cloning: A Laboratory Manual, Second Edition, Cold Spring Harbor Laboratory Press, Cold Spring Harbor, N.Y. (1989).

As known to a person skilled in the art enables the use of recombinant DNA technology the production of numerous derivatives of a nucleic acid and/or polypeptide. Such derivatives can, for example, be modified in one individual or several positions by substitution, alteration, exchange, deletion, or insertion. The modification or derivatization can, for example, be carried out by means of site directed mutagenesis. Such modifications can easily be carried out by a person skilled in the art (see e.g. Sambrook, J., et al., Molecular Cloning: A laboratory manual (1999) Cold Spring Harbor Laboratory Press, New York, USA). The use of recombinant technology enables a person skilled in the art to transform various host cells with heterologous nucleic acid(s). Although the transcription and translation, i.e. expression, machinery of different cells use the same elements, cells belonging to different species may have among other things a different so-called codon usage. Thereby identical polypeptides (with respect to amino acid sequence) may be encoded by different nucleic acid(s). Also, due to the degeneracy of the genetic code, different nucleic acids may encode the same polypeptide.

The use of recombinant DNA technology enables the production of numerous derivatives of a nucleic acid and/or polypeptide. Such derivatives can, for example, be modified in one individual or several positions by substitution, alteration, exchange, deletion, or insertion. The modification or derivatization can, for example, be carried out by means of site directed mutagenesis. Such modifications can easily be carried out by a person skilled in the art (see e.g. Sambrook, J., et al., Molecular Cloning: A laboratory manual (1999) Cold Spring Harbor Laboratory Press, New York, USA; Hames, B.D., and Higgins, S.G., Nucleic acid hybridization - a practical approach (1985) IRL Press, Oxford, England).

The use of recombinant technology enables the transformation of various host cells with heterologous nucleic acid(s). Although the transcription and translation, i.e. expression, machinery of different cells use the same elements, cells belonging to different species may have among other things a different so-called codon usage. Thereby identical polypeptides (with respect to amino acid sequence) may be encoded by different nucleic acid(s). Also, due to the degeneracy of the genetic code, different nucleic acids may encode the same polypeptide.

Within the scope of the present invention, transfected cells may be obtained with substantially any kind of transfection method known in the art. For example, the nucleic acid may be introduced into the cells by means of electroporation or microinjection. Alternatively, lipofection reagents such as FuGENE 6 (Roche Diagnostics GmbH, Germany), X-tremeGENE (Roche Diagnostics GmbH, Germany), and LipofectAmine (Invitrogen Corp., USA) may be used. Still alternatively, the nucleic acid may be introduced into the cell by appropriate viral vector systems based on retroviruses, lentiviruses, adenoviruses, or adeno-associated viruses (Singer, O., Proc. Natl. Acad. Sci. USA 101 (2004) 5313-5314).

### DEFINITIONS

The term "antibody" encompasses the various forms of antibody structures including whole antibodies and antibody fragments. The antibody according to the invention is preferably a human antibody, a humanized antibody, a chimeric antibody, a T cell antigen depleted antibody (WO 98/33523, WO 98/52976, and WO 00/34317). Genetic engineering of antibodies is e.g. described in Morrison, S.L., et al., Proc. Natl. Acad. Sci. USA 81 (1984) 6851-6855; US Patent Nos. 5,202,238 and 5,204,244; Riechmann, L., et al., Nature 332 (1988) 323-327; Neuberger, M.S., et al., Nature 314 (1985) 268-270; Lonberg, N., Nat. Biotechnol. 23 (2005) 1117-1125.

The term "antibody" refers to a protein consisting of one or more polypeptide(s) substantially encoded by antibody genes. The recognized antibody genes include the different constant region genes as well as the myriad antibody variable region genes. Antibodies may exist in a variety of formats, including, for example, Fv, Fab, and F(ab)₂ as well as single chains (scFv) or diabodies (e.g. Huston, J.S., et al., Proc. Natl. Acad. Sci. USA 85 (1988) 5879-5883; Bird, R.E., et al., Science 242 (1988) 423-426; in general, Hood et al., Immunology, Benjamin N.Y., 2nd edition (1984); and Hunkapiller, T. and Hood, L., Nature 323 (1986) 15-16).

An antibody in general comprises two so called light chain polypeptides (light chain) and two so called heavy chain polypeptides (heavy chain). Each of the heavy and light chain polypeptides contains a variable domain (variable region) (generally the amino terminal portion of the polypeptide chain) comprising binding regions that are able to interact with an antigen. Each of the heavy and light chain polypeptides comprises a constant region (generally the carboxyl terminal portion). The constant region of the heavy chain mediates the binding of the antibody i) to cells bearing a Fc gamma receptor (FcγR), such as phagocytic cells, or ii) to cells bearing the neonatal Fc receptor (FcRn) also known as Brambell receptor. It also mediates the binding to some factors including factors of the classical complement system such as component (C1q).

The variable domain of an antibody's light or heavy chain in turn comprises different segments, i.e. four framework regions (FR) and three hypervariable regions (CDR).

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g. scFv); and multispecific antibodies formed from antibody fragments.

The term "biologically active polypeptide" as used herein refers to an organic molecule, e.g. a biological macromolecule such as a peptide, protein, glycoprotein, nucleoprotein, mucoprotein, lipoprotein, synthetic polypeptide or protein, that causes a biological effect when administered in or to artificial biological systems, such as bioassays using cell lines and viruses, or in vivo to an animal, including but not limited to birds or mammals, including humans. This biological effect can be but is not limited to enzyme inhibition or activation, binding to a receptor or a ligand, either at the binding site or circumferential, signal triggering or signal modulation. Biologically active molecules are without limitation for example immunoglobulins, or hormones, or cytokines, or growth factors, or receptor ligands, or agonists or antagonists, or cytotoxic agents, or antiviral agents, or imaging agents, or enzyme inhibitors, enzyme activators or enzyme activity modulators such as allosteric substances. In a preferred embodiment the heterologous polypeptide is an immunoglobulin, immunoglobulin conjugate, or an antifusogenic peptide.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called α, β, ε, γ and µ, respectively.

The term "enzymatic cleavage site" denotes a sequence of amino acid residues connected to each other via peptides bonds that can specifically be cleaved by a protease. In one embodiment the protease is IgA-protease, Granzyme B, Tev protease, PreScission protease, Thrombin, Faktor10a, Ides protease, or Enterokinase.

The term "IgA-protease" denotes a protease derived from Neisseria gonorrhoeae with a recognition site comprising one of the following sequences wherein "↓" denotes the position of the cleaved bond:

| | |
|---|---|
| Pro-Ala-Pro ↓ Ser-Pro | (SEQ ID NO: 08), |
| Pro-Pro ↓ Ser-Pro | (SEQ ID NO: 09), |
| Pro-Pro ↓ Ala-Pro | (SEQ ID NO: 10), |
| Pro-Pro ↓ Thr-Pro | (SEQ ID NO: 11), |
| Pro-Pro ↓ Gly-Pro | (SEQ ID NO: 12), |
| Pro-Arg-Pro-Pro ↓ Thr-Pro | (SEQ ID NO: 13), |
| Val-Val-Ala-Pro-Pro ↓ Ala-Pro | (SEQ ID NO: 14), |
| Val-Val-Ala-Pro-Pro ↓ Ser-Pro | (SEQ ID NO: 15), |
| Val-Val-Ala-Pro-Pro ↓ Thr-Pro | (SEQ ID NO: 16), |
| Val-Val-Ala-Pro-Pro ↓ Gly-Pro | (SEQ ID NO: 17), |
| Pro-Arg-Pro-Pro ↓ Thr-Pro | (SEQ ID NO: 18), |
| Ala-Pro-Pro-Ala ↓ Ala-Pro | (SEQ ID NO: 19), |
| Pro-Arg-Pro-Pro ↓ Ala-Pro | (SEQ ID NO: 20), |
| Pro-Arg-Pro-Pro ↓ Ser-Pro | (SEQ ID NO: 21), |
| Pro-Arg-Pro-Pro ↓ Gly-Pro | (SEQ ID NO: 22). |

As a result of the specific cleavage two amino acids - as first alanine or glycine or serine or proline and as second proline - are maintained at the N-terminus of the N-terminal polypeptide of the fusion polypeptide.

The term "expression" as used herein refers to transcription and/or translation processes occurring within a cell. The level of transcription of a nucleic acid sequence of interest in a cell can be determined on the basis of the amount of corresponding mRNA that is present in the cell. For example, mRNA transcribed from a sequence of interest can be quantitated by RT-PCR or by Northern hybridization (see Sambrook et al., 1989, supra). Polypeptides encoded by a nucleic acid of interest can be quantitated by various methods, e.g. by ELISA, by assaying for the biological activity of the polypeptide, or by employing assays that are independent of such activity, such as Western blotting or radioimmunoassay, using immunoglobulins that recognize and bind to the polypeptide (see Sambrook et al., 1989, supra).

An "expression cassette" refers to a construct that contains the necessary regulatory elements, such as promoter and polyadenylation site, for expression of at least the contained nucleic acid in a cell.

The term "expression machinery" as used within the current invention denotes the sum of the enzymes, cofactors, etc. of a cell that is involved in the steps beginning with the transcription step of a nucleic acid or gene (i.e. also called "gene expression machinery") to the post-translational modification of the polypeptide encoded by the nucleic acid. The expression machinery e.g. comprises the steps of transcription of DNA into pre-mRNA, pre-mRNA splicing to mature mRNA, translation into a polypeptide of the mRNA, and post translational modification of the polypeptide.

An "expression vector" is a nucleic acid providing all required elements for the expression of the comprised structural gene(s) in a host cell. Typically, an expression vector comprises a prokaryotic plasmid propagation unit, e.g. for E. coli, comprising an origin of replication, and a selectable marker, an eukaryotic selection marker, and one or more expression cassettes for the expression of the structural gene(s) of interest each comprising a promoter, a structural gene, and a transcription terminator including a polyadenylation signal. Gene expression is usually placed under the control of a promoter, and such a structural gene is said to be "operably linked to" the promoter. Similarly, a regulatory element and a core promoter are operably linked if the regulatory element modulates the activity of the core promoter.

The terms "full length antibody", "intact antibody," and "whole antibody" are used herein interchangeably to refer to an antibody having a structure substantially similar to a native antibody structure or having heavy chains that contain an Fc region as defined herein.

The terms "host cell", "host cell line", and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman, S. et al., J. Chrom. B 848 (2007) 79-87.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "linker polypeptide" denotes peptide linkers of natural and/or synthetic origin. They consist of a linear amino acid chain wherein the 20 naturally occurring amino acids are the monomeric building blocks. The chain has a length of from 1 to 50 amino acids, in one embodiment between 1 and 28 amino acids, in one embodiment between 3 and 25 amino acids. The linker may contain repetitive amino acid sequences or sequences of naturally occurring polypeptides, such as polypeptides with a hinge-function. The linker has the function to ensure that the individual entities of a fusion polypeptide can perform their biological activity by allowing the entities to fold correctly and to be presented properly. In one embodiment the linker polypeptide is a "synthetic linker polypeptide" that is designated to be rich in glycine, glutamine, and/or serine residues. These residues are arranged e.g. in small repetitive units of up to five amino acids, such as GGGGS, QQQQG, or SSSSG. This small repetitive unit may be repeated for two to five times to form a multimeric unit. At the amino- and/or carboxy-terminal ends of the multimeric unit up to six additional arbitrary, naturally occurring amino acids may be added. Other synthetic peptidic linkers are composed of a single amino acid, which is repeated between 10 to 20 times and may comprise at the amino- and/or carboxy-terminal end up to six additional arbitrary, naturally occurring amino acids, such as e.g. serine in the linker GS₁₅G. All linker polypeptides can be encoded by a nucleic acid molecule and therefore can be recombinantly expressed. As the linker polypeptides are themselves polypeptides, the entities of the fusion polypeptide are connected to the linker via a peptide bond that is formed between two amino acids. In one embodiment the polypeptide linker has an acid sequence selected from the group comprising (G₃S)₃, (G₃S)₄, (G₃S)₅, (G₃S)₆, (G₄S)₃, (G₄S)₄, (G₄S)₅, (G₅S)₂, (G₅S)₃, and (G₅S)₄.

The term "mammalian cell" denotes cells which are used for the expression of nucleic acids for the recombinant production of a polypeptide. In one embodiment the mammalian cell is a CHO cell (e.g. CHO K1, CHO DG44), or a BHK cell, or a NS0 cell, or a SP2/0 cell, or a HEK 293 cell, or a HEK 293 EBNA cell, or a PER.C6^{®} cell, or a COS cells. In one embodiment the cell is a CHO cell, or a HEK 293 cell. As used herein, the expression "cell" includes the subject cell and its progeny. Thus, the term "mammalian cell" includes the primary transfected cell and cultures including the progeny cells derived there from without regard to the number of transfers. It is also understood that all progeny may not be precisely identical in DNA content, due to deliberate or inadvertent mutations. Variant progeny that have the same function or biological activity as the originally transformed cell are included.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "nucleic acid" as used herein, refers to a polymeric molecule consisting of individual nucleotides (also called bases) a, c, g, and t (or u in RNA), for example to DNA, RNA, or modifications thereof. This polynucleotide molecule can be a naturally occurring polynucleotide molecule or a synthetic polynucleotide molecule or a combination of one or more naturally occurring polynucleotide molecules with one or more synthetic polynucleotide molecules. Also encompassed by this definition are naturally occurring polynucleotide molecules in which one or more nucleotides are changed (e.g. by mutagenesis), deleted, or added. A nucleic acid can either be isolated, or integrated in another nucleic acid, e.g. in an expression cassette, a plasmid, or the chromosome of a host cell. A nucleic acid is likewise characterized by its nucleic acid sequence consisting of individual nucleotides.

To a person skilled in the art procedures and methods are well known to convert an amino acid sequence, e.g. of a polypeptide, into a corresponding nucleic acid sequence encoding this amino acid sequence. Therefore, a nucleic acid is characterized by its nucleic acid sequence consisting of individual nucleotides and likewise by the amino acid sequence of a polypeptide encoded thereby.

A "nucleic acid" denotes a naturally occurring or partially or fully non-naturally occurring nucleic acid encoding a polypeptide which can be produced recombinantly. The nucleic acid can be build up of DNA-fragments which are either isolated or synthesized by chemical means. The nucleic acid can be integrated into another nucleic acid, e.g. in an expression vector or the genome/chromosome of a mammalian cell. Vectors include shuttle and expression vectors. Typically, the vectors will also comprise a prokaryotic propagation unit comprising an origin of replication (e.g. the ColE1 origin of replication) and a selectable marker (e.g. ampicillin or tetracycline resistance gene), for replication and selection, respectively, of the vector in prokaryotes.

A "polypeptide" is a polymer consisting of amino acids joined by peptide bonds, whether produced naturally or synthetically. Polypeptides of less than about 20 amino acid residues may be referred to as "peptides", whereas molecules consisting of two or more polypeptides or comprising one polypeptide of more than 100 amino acid residues may be referred to as "proteins". A polypeptide may also comprise non-amino acid components, such as carbohydrate groups, metal ions, or carboxylic acid esters. The non-amino acid components may be added by the cell, in which the polypeptide is expressed, and may vary with the type of cell. Polypeptides are defined herein in terms of their amino acid backbone structure or the nucleic acid encoding the same. Additions such as carbohydrate groups are generally not specified, but may be present nonetheless.

The term "vector" as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors".

### RECOMBINANT PRODUCTION OF POLYPEPTIDES

Suitable host cells for cloning and/or expression/secretion of polypeptide-encoding vectors include prokaryotic and eukaryotic cells described herein. For example, polypeptides may be produced in bacteria, in particular when glycosylation and Fc effector function are not needed (see, e.g., US 5,648,237, US 5,789,199, and US 5,840,523, Charlton, Methods in Molecular Biology 248 (2003) 245-254 (B.K.C. Lo, (ed.), Humana Press, Totowa, NJ), describing expression of antibody fragments in E. coli.). After expression, the polypeptide may be isolated from the bacterial cell paste in a soluble fraction or may be isolated from the insoluble fraction, so-called inclusion bodies which can be solubilized and the polypeptide be refolded to bioactive forms. Thereafter the polypeptide can be further purified.

In addition to prokaryotes, eukaryotic microbes such as filamentous fungi or yeasts are suitable cloning or expression hosts for polypeptide-encoding vectors, including fungi and yeast strains whose glycosylation pathways have been "humanized", resulting in the production of a polypeptide with a partially or fully human glycosylation pattern (see e.g. Gerngross, Nat. Biotech. 22 (2004) 1409-1414, and Li, et al., Nat. Biotech. 24 (2006) 210-215).

Suitable host cells for the expression of glycosylated polypeptides are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells.

Plant cell cultures can also be utilized as hosts (see, e.g., US 5,959,177, US 6,040,498, US 6,420,548, US 7,125,978, and US 6,417,429 (describing PLANTIBODIES^{™} technology for producing antibodies in transgenic plants)).

Vertebrate cells may also be used as hosts. For example, mammalian cell lines that are adapted to grow in suspension culture may be useful. Other examples of useful mammalian host cell lines are the COS-7 cell line (monkey kidney CV1 cell transformed by SV40; the HEK293 cell line (human embryonic kidney) BHK cell line (baby hamster kidney); the TM4 mouse sertoli cell line (TM4 cells as described, e.g., in Mather, Biol. Reprod. 23 (1980) 243-251); the CV1 cell line (monkey kidney cell); the VERO-76 cell line (African green monkey kidney cell); the HELA cell line (human cervical carcinoma cell); the MDCK cell line (canine kidney cell); the BRL-3A cell line (buffalo rat liver cell); the W138 cell line (human lung cell); the HepG2 cell line (human liver cell); the MMT 060562 cell line (mouse mammary tumor cell); the TRI cell line, as described, e.g., in Mather, et al., Annals N.Y. Acad. Sci. 383 (1982) 44-68; the MRC5 cell line; and FS4 cell-s line. Other useful mammalian host cell lines include the CHO cell line (Chinese hamster ovary cell), including DHFR negative CHO cell lines (Urlaub, et al., Proc. Natl. Acad. Sci. USA 77 (1980) 4216), and myeloma cell lines such as Y0, NS0 and Sp2/0 cell line. For a review of certain mammalian host cell lines suitable for polypeptide production, see, e.g., Yazaki, and Wu, Methods in Molecular Biology, Antibody Engineering 248 (2004) 255-268 (B.K.C. Lo, (ed.), Humana Press, Totowa, NJ).

### THE METHOD AS REPORTED HEREIN

One aspect as reported herein is a method for the recombinant production of a polypeptide in a mammalian cell comprising the following steps:
- cultivating a mammalian cell expressing the polypeptide as fusion polypeptide with human neprilysin whereby the neprilysin is fused to the C-terminus of the polypeptide, and
- recovering the polypeptide from the cell or the cultivation medium and thereby producing the polypeptide.

One aspect as reported herein is the use of human neprilysin fused to the C-terminus of a polypeptide for increasing the expression yield of the polypeptide in a mammalian cell.

It has been found that by fusing human neprilysin or a fragment thereof to the C-terminus of a polypeptide the recombinant yield of the polypeptide can be increased when expressed using a mammalian cell.

In the following the method and use as reported herein is exemplified with an antibody comprising a full length antibody directed to Abeta (MAB31). To this antibody different short and long polypeptides are fused at the C-terminus of the heavy chains.

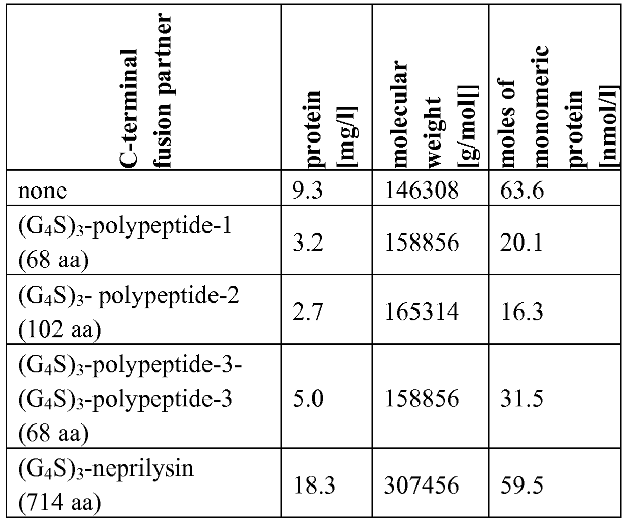

It can be seen that fusion of the neprilysin residue to the antibody heavy chain increased the obtained expression yield.

The following examples, figures and sequences are provided to aid the understanding of the present invention, the true scope of which is set forth in the appended claims. It is understood that modifications can be made in the procedures set forth without departing from the spirit of the invention.

Although the foregoing invention has been described in some detail by way of illustration and example for purposes of clarity of understanding, the descriptions and examples should not be construed as limiting the scope of the invention.

### EXAMPLES

### Polypeptides used in the examples

Trifunctional antibody (TriGant) comprises a full length antibody directed to Abeta (MAB31), a single Fab directed to the transferrin receptor and neprilysin. The sequence of the heavy chains and the variable chains are as follows:
mAb31-IgG1 without C-terminal GK 
QVELVESGGGLVQPGGSLRLSCAASGFTFSSYAMSWVRQAPGKGLEWVSA
INASGTRTYYADSVKGRFTISRDNSKNTLYLQMNSLRAEDTAVYYCARGK
GNTHKPYGYVRYFDVWGQGTLVTVSSASTKGPSVFPLAPSSKSTSGGTAA
 
LGCLVKDYFPEPVTVSWNSGALTSGVHTFPAVLQSSGLYSLSSVVTVPSS
SLGTQTYICNVNHKPSNTKVDKKVEPKSCDKTHTCPPCPAPELLGGPSVF
LFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKG
QPREPQVYTLPPSRDELTKNQVSLTCLVKGFYPSDIAVEWESNGQPENNY
KTTPPVLDSDGSFFLYSKLTVDKSRWQQGNVFSCSVMHEALHNHYTQKSL
SLSP
 (SEQ ID NO: 0 1)

Composition of SEQ ID NO: 01:
- anti-Abeta antibody mAb 31 (= Gantenerumab (INN)) human IgG1 heavy
   chain without C-terminal Gly and Lys.
   mAb31-IgG1-knob without C-terminal GK 
QVELVESGGGLVQPGGSLRLSCAASGFTFSSYAMSWVRQAPGKGLEWVSA
INASGTRTYYADSVKGRFTISRDNSKNTLYLQMNSLRAEDTAVYYCARGK
GNTHKPYGYVRYFDVWGQGTLVTVSSASTKGPSVFPLAPSSKSTSGGTAA
LGCLVKDYFPEPVTVSWNSGALTSGVHTFPAVLQSSGLYSLSSVVTVPSS
SLGTQTYICNVNHKPSNTKVDKKVEPKSCDKTHTCPPCPAPELLGGPSVF
LFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKG
QPREPQVYTLPPCRDELTKNQVSLWCLVKGFYPSDIAVEWESNGQPENNY
KTTPPVLDSDGSFFLYSKLTVDKSRWQQGNVFSCSVMHEALHNHYTQKSL
SLSP
 (SEQ ID NO: 02)

Composition of SEQ ID NO: 02:
- mAb31 human IgG1 heavy chain without C-terminal Gly and Lys with knob mutations (T366W and S354C, numbering of the amino acid residues according to Kabat (Kabat et al. 1991, Sequences of Proteins of immunological Interest, U.S. Department of Public Health, Bethesda, Md.)).
   mAb31-IgG 1-neprilysin (SEQ ID NO: 03)

Composition of SEQ ID NO: 03:
- mAb31 human IgG1 heavy chain without C-terminal Gly and Lys
- glycine-serine-linker
- amino acid residues 52 - 750 ofNeprilysin (SEQ ID NO: 04).
   mAb31-IgG1-KNOB-SS_G4S-4_VL-8D3-CK_G4S-6-GG_VH-8D3-CH1 
QVELVESGGGLVQPGGSLRLSCAASGFTFSSYAMSWVRQAPGKGLEWVSA
INASGTRTYYADSVKGRFTISRDNSKNTLYLQMNSLRAEDTAVYYCARGK
GNTHKPYGYVRYFDVWGQGTLVTVSSASTKGPSVFPLAPSSKSTSGGTAA
LGCLVKDYFPEPVTVSWNSGALTSGVHTFPAVLQSSGLYSLSSVVTVPSS
SLGTQTYICNVNHKPSNTKVDKKVEPKSCDKTHTCPPCPAPELLGGPSVF
 
LFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKG
QPREPQVYTLPPCRDELTKNQVSLWCLVKGFYPSDIAVEWESNGQPENNY
KTTPPVLDSDGSFFLYSKLTVDKSRWQQGNVFSCSVMHEALHNHYTQKSL
SLSPGGGSGGGGSGGGGSGGGGSDIQMTQSPASLSASLEEIVTITCQASQ
DIGNWLAWYQQKPGKSPQLLIYGATSLADGVPSRFSGSRSGTQFSLKISR
VQVEDIGIYYCLQAYNTPWTFGGGTKVEIKRTVAAPSVFIFPPSDEQLKS
GTASVVCLLNNFYPREAKVQWKVDNALQSGNSQESVTEQDSKDSTYSLSS
TLTLSKADYEKHKVYACEVTHQGLSSPVTKSFNRGECGGGGSGGGGSGGG
GSGGGGSGGGGSGGGGSGGEVQLVESGGGLVQPGNSLTLSCVASGFTFSN
YGMHWIRQAPKKGLEWIAMIYYDSSKMNYADTVKGRFTISRDNSKNTLYL
EMNSLRSEDTAMYYCAVPTSHYVVDVWGQGVSVTVSSASTKGPSVFPLAP
SSKSTSGGTAALGCLVKDYFPEPVTVSWNSGALTSGVHTFPAVLQSSGLY
SLSSVVTVPSSSLGTQTYICNVNHKPSNTKVDKKVEPKSC
 (SEQ ID NO: 05)

Composition of SEQ ID NO: 05:
- Mab31 human IgG1 heavy chain without C-terminal Lys
- glycine-serine-linker
- Variable light chain domain (VL) variant (L596V and L598I) of the mouse 8D3 anti-transferrin antibody (Boado, R.J., et al., Biotechnol. Bioeng. 102 (2009) 1251-1258)
- human C-kappa light chain
- glycine-serine-linker
- variable heavy chain domain (VH) of the mouse 8D3 anti-transferrin antibody (Boado, R.J., et al., Biotechnol. Bioeng. 102 (2009) 1251-1258)
- human IgG1 CH3 heavy chain domain.
   mAb31_8D3_HC-HOLE_NEPRI 
QVELVESGGGLVQPGGSLRLSCAASGFTFSSYAMSWVRQAPGKGLEWVSA
INASGTRTYYADSVKGRFTISRDNSKNTLYLQMNSLRAEDTAVYYCARGK
GNTHKPYGYVRYFDVWGQGTLVTVSSASTKGPSVFPLAPSSKSTSGGTAA
 
LGCLVKDYFPEPVTVSWNSGALTSGVHTFPAVLQSSGLYSLSSVVTVPSS
SLGTQTYICNVNHKPSNTKVDKKVEPKSCDKTHTCPPCPAPELLGGPSVF
LFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKG
QPREPQVCTLPPSRDELTKNQVSLSCAVKGFYPSDIAVEWESNGQPENNY
KTTPPVLDSDGSFFLVSKLTVDKSRWQQGNVFSCSVMHEALHNHYTQKSL
SLSPGGGGSGGGGSGGGGSYDDGICKSSDCIKSAARLIQNMDATTEPCTD
FFKYACGGWLKRNVIPETSSRYGNFDILRDELEVVLKDVLQEPKTEDIVA
VQKAKALYRSCINESAIDSRGGEPLLKLLPDIYGWPVATENWEQKYGASW
TAEKAIAQLNSKYGKKVLINLFVGTDDKNSVNHVIHIDQPRLGLPSRDYY
ECTGIYKEACTAYVDFMISVARLIRQEERLPIDENQLALEMNKVMELEKE
IANATAKPEDRNDPMLLYNKMTLAQIQNNFSLEINGKPFSWLNFTNEIMS
TVNISITNEEDVVVYAPEYLTKLKPILTKYSARDLQNLMSWRFIMDLVSS
LSRTYKESRNAFRKALYGTTSETATWRRCANYVNGNMENAVGRLYVEAAF
AGESKHVVEDLIAQIREVFIQTLDDLTWMDAETKKRAEEKALAIKERIGY
PDDIVSNDNKLNNEYLELNYKEDEYFENIIQNLKFSQSKQLKKLREKVDK
DEWISGAAVVNAFYSSGRNQIVFPAGILQPPFFSAQQSNSLNYGGIGMVI
GHEITHGFDDNGRNFNKDGDLVDWWTQQSASNFKEQSQCMVYQYGNFSWD
LAGGQHLNGINTLGENIADNGGLGQAYRAYQNYIKKNGEEKLLPGLDLNH
KQLFFLNFAQVWCGTYRPEYAVNSIKTDVHSPGNFRIIGTLQNSAEFSEA
FHCRKNSYMNPEKKCRVW
 (SEQ ID NO: 06)

Composition of SEQ ID NO: 06:
- mAb31 human IgG1 heavy chain without C-terminal Lys and Gly with hole mutations (mutations T366S, L368A, Y407V, and Y349C, numbering according to Kabat)
- glycine-serine-linker
- amino acid residues 52 - 750 ofNeprilysin (SEQ ID NO: 04).
   mAb31-LC 
DIVLTQSPATLSLSPGERATLSCRASQSVSSSYLAWYQQKPGQAPRLLIY
GASSRATGVPARFSGSGSGTDFTLTISSLEPEDFATYYCLQIYNMPITFG
QGTKVEIKRTVAAPSVFIFPPSDEQLKSGTASVVCLLNNFYPREAKVQWK
VDNALQSGNSQESVTEQDSKDSTYSLSSTLTLSKADYEKHKVYACEVTHQ
 GLSSPVTKSFNRGEC
   (SEQ ID NO: 07)

Composition of SEQ ID NO: 07:
- mAb 31 kappa light chain.

### Example 1

### Recombinant production

### DNA preparation

500 ml or 5 L of overnight bacterial LB culture were harvested and plasmid DNA was extracted according to the manufacturer's protocol (High speed Maxi kit, Qiagen, Cat. No. 12663). The resulting plasmid DNA was eluted in 1 ml TE buffer and DNA concentration was determined by spectrophotometric measurement (Epoch, BioTek).

### Expression plasmids:

Expression plasmids comprise expression cassettes for the expression of the heavy and light chains were separately assembled in mammalian cell expression vectors.

General information regarding the nucleotide sequences of human light and heavy chains from which the codon usage can be deduced is given in: Kabat, E.A., et al., Sequences of Proteins of Immunological Interest, 5th ed., Public Health Service, National Institutes of Health, Bethesda, MD (1991), NIH Publication No 91-3242.
a) antibody without neprilysin:
   The transcription unit of the κ-light chain is composed of the following elements:
      - the immediate early enhancer and promoter from the human cytomegalovirus (hCMV) including a 5'UTR of a human light chain germline gene,
      - the light chain variable region including the signal peptide sequence encoding genomic DNA segment (L1, signal-sequence-intron, L2) of a human germline gene,
      - the human κ-light gene constant region including the mouse κ-light gene intron 2, and
      - the SV40 polyadenylation ("poly A") signal sequence.

The transcription unit of the γ1-heavy chain is composed of the following elements:
- the immediate early enhancer and promoter from the human cytomegalovirus (HCMV) including the 5'-UTR of a human heavy chain germline gene,
- the γ1-chain variable region including the signal peptide sequence encoding genomic DNA segment (L1, signal-sequence-intron, L2) of a human germline gene,
- the genomic human γl-heavy chain gene constant region including the mouse heavy chain intron 2;
- the SV40 polyadenylation ("poly A") signal sequence.

Further the plasmid contains
- a neomycin resistance gene,
- an origin of replication from the vector pUC18 which allows replication of this plasmid in E. coli, and
- a β-lactamase gene which confers ampicillin resistance in E. coli.
   b) antibody with neprilysin fused to the C-terminus of the heavy chain:
      The expression plasmid for the light chain comprises
         - the transcription unit of the κ-light chain composed of the following elements:
            - the immediate early enhancer and promoter from the human cytomegalovirus (hCMV) including a 5'UTR of a human light chain germline gene,
            - the light chain variable region including the signal peptide sequence encoding genomic DNA segment (L1, signal-sequence-intron, L2) of a murine germline gene,
            - the human κ-light gene constant region including the mouse κ-light gene intron 2, and
            - the BGH polyadenylation ("poly A") signal sequence;
         - a neomycin resistance gene,
         - an origin of replication from the vector pUC18 which allows replication of this plasmid in E. coli, and
         - a β-lactamase gene which confers ampicillin resistance in E. coli.

The expression plasmid for the heavy chain comprises
- the transcription unit of the γl-heavy chain composed of the following elements:
   - the immediate early enhancer and promoter from the human cytomegalovirus (HCMV) including the 5'-UTR of a human heavy chain germline gene,
   - the γ1-chain variable region including the signal peptide sequence encoding genomic DNA segment (L1, signal-sequence-intron, L2) of a human germline gene,
   - the genomic human γl-heavy chain gene constant region including the mouse heavy chain intron 2;
   - a neprilysin encoding nucleic acid,
   - the BGH polyadenylation ("poly A") signal sequence;
- an origin of replication from the vector pUC18 which allows replication of this plasmid in E. coli, and
- a β-lactamase gene which confers ampicillin resistance in E. coli.

### Transfection:

HEK293 cells were diluted to 8 x 10⁵ cells/ml the day before transfection. About 1 to 1.6 x 10⁶ cells/ml were transfected according to the manufacturer's protocol. For a final transfection volume of 1000 ml, 1000 µg DNA were diluted to a final volume of 50 ml with Opti-MEM^{®} I Reduced Serum Medium (Gibco, Cat. No. 31985070). Two microliter of 293fectin™Reagent (Invitrogen, Cat. No. 12347019) per 1 µg DNA were equally diluted to a final volume of 1 ml with Opti-MEM^{®} medium and incubated for 5 minutes. After incubation the diluted DNA was added to the diluted 293fectin^{™}Reagent, gently mixed, incubated for another 20-30 minutes and afterwards drop wise pipetted to 950 ml of the HEK293 cell suspension to obtain a final volume of 1000 ml. The cells were incubated under cell culture condition (37 °C, 8 % CO₂, 80 % humidity) on an orbital shaker rotating at 125 rpm and harvested after 72 hours. The harvest was centrifuged for 10 minutes at 1000 rpm followed by 10 minutes at 3000 rpm and filtered through a 22 µm sterile filter (Millipore, Cat. No. SCGPU05RE).

### Purification:

Cells were removed from culture medium by centrifugation. Complexes were purified from supernatants by Protein A affinity chromatography (MabSelect-Sepharose on an ÄKTA-Avant). Eluted complexes were concentrated with Amicon centrifugation tubes to a protein concentration of 3 mg/ml. An aliquot was analyzed on a size exclusion chromatography (HPLC TSKgel GFC300 Sys89). Preparative SEC on a Superdex 200 was performed to remove aggregates and to buffer the fusion proteins in 20 mM histidine, 140 mM NaCl, pH 6.0. Eluted complexes were concentrated with Amicon centrifugation tube to a protein concentration of 1 mg/ml and sterile filtered (0.2 µm pore size).

### Analytics:

Complex samples were analyzed by OD280 using a UV spectrophotometer to determine the protein concentration in solution. The extinction coefficient required for this was calculated from the amino acid sequence according to Pace (Pace et al., Protein Science 4 (1995) 2411-2423). Size-exclusion chromatography (SE-HPLC) was performed on TSK-Ge1300SWXL or Superdex 200 columns with a 0.2 M potassium phosphate buffer, comprising 0.25 M KCI, pH 7.0 as mobile phase in order to determine the content of monomeric, aggregated and degraded species in the samples. Sodium dodecyl sulfate (SDS) polyacrylamide gel electrophoresis (reducing and non-reducing) was performed to analyze the purity of the complex preparations with regard to product-related degradation products and unrelated impurities. Electrospray ionization mass spectrometry (ESI-MS) was performed with reduced (TCEP) and deglycosylated (N-glycosidase F) samples to confirm the correct mass/identity of each chain and detect chemical modifications. ESI-MS of the deglycosylated samples was carried out to analyze the nature and quality of the fully assembled protein and detect potential product-related side products.

### Results:

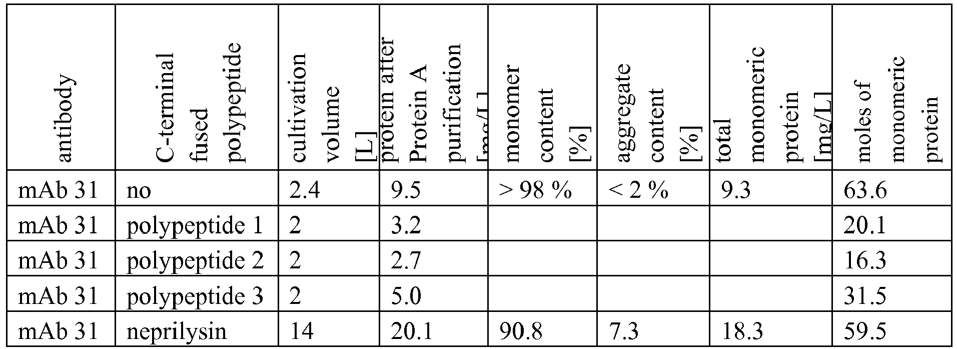

It can be seen that fusion of the neprilysin residue to the antibody heavy chain increased the expression yield in mg/L.

### Example 2

### Determination of binding to Aβ1-40 fibers in vitro by ELISA

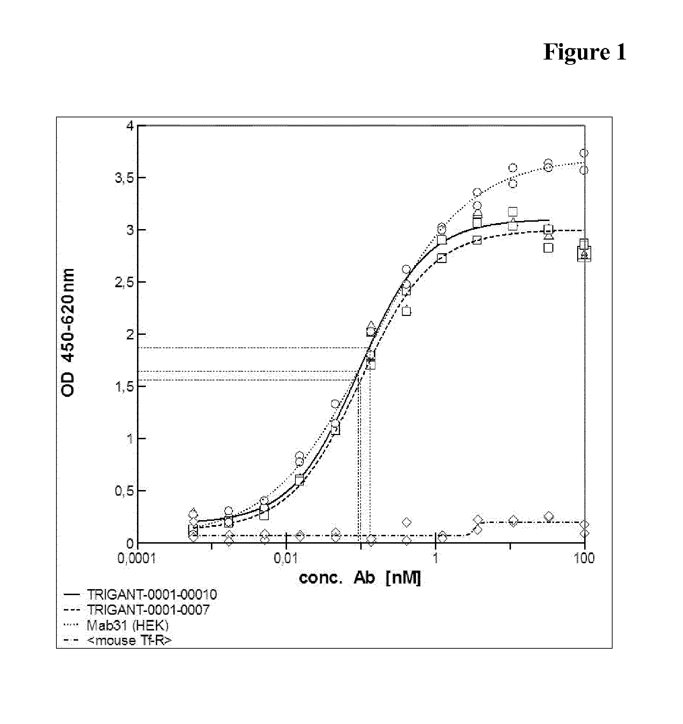

Binding of fusion polypeptide to fibrillar Aβ was measured by an ELISA assay. Briefly, Aβ(1-40) was coated at 7 µg/mL in PBS onto Maxisorb plates for 3 days at 37°C to produce fibrillar Abeta, then dried for 3 h at RT. The plate was blocked with 1% CroteinC and 0.1% RSA in PBS (blocking buffer) for 1 h at RT, then washed once with wash buffer. Fusion polypeptides or controls were added at concentrations up to 100 nM in blocking buffer and incubated at 4°C overnight. After 4 wash steps, constructs were detected by addition of anti-human-IgG-HRP (Jackson Immunoresearch) at 1:10,000 dilution in blocking buffer (1 RT), followed by 6 washes and incubation in TMB (Sigma). Absorbance was read out at 450 nm after stopping color development with 1 N HCl (see Figure 1).

### Example 3

### Determination of binding to murine transferrin receptor in vitro

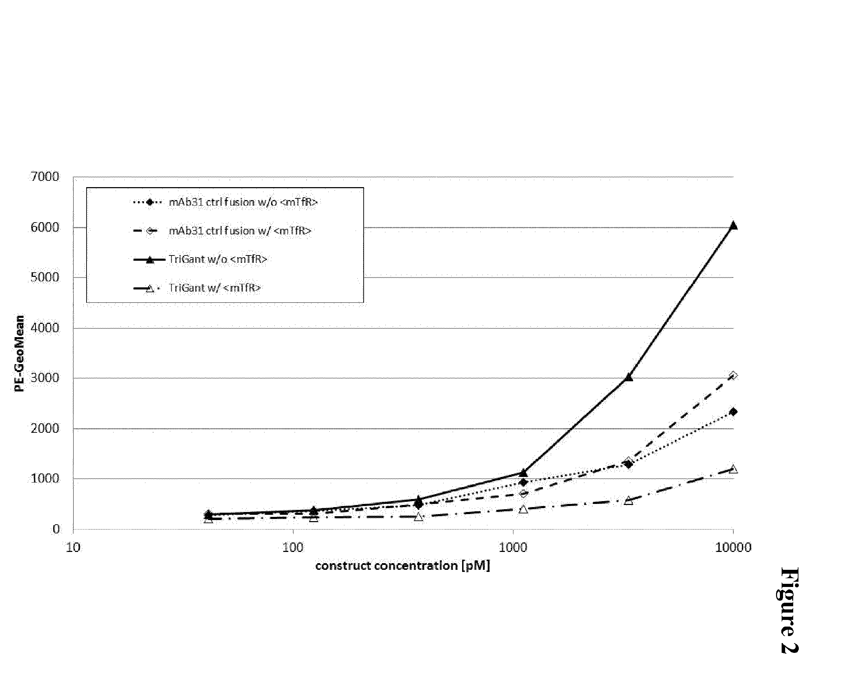

Binding of fusion polypeptide to murine transferrin receptor was tested by FACS analysis on mouse X63.AG8-563 myeloma cells. As Aβ antibody mAb31(HEK) showed a certain tendency to non-specifically bind to Ag8 cells, specific binding was quantified by co-incubation with a 20fold excess of anti-mouse-TfR antibody. Cells were harvested by centrifugation, washed once with PBS and 5 x 10⁴ cells incubated with a 1.5 pM to 10 nM dilution series of the polypeptide fusions with or without addition of 200 nM anti-mouse TfR antibody in 100 µL RPMI/10% FCS for 1.5 h on ice. After 2 washes with RPMI/10% FCS, cells were incubated with goat-anti-human IgG coupled to Phycoerythrin (Jackson Immunoresearch) at a dilution of 1:600 in RPMI/19% FCS for 1.5 h on ice. Cells were again washed, resuspended in RPMI/10% FCS and Phycoerythrin fluorescence measured on a FACS-Array instrument (Becton-Dickinson) (see Figure 2).

### Example 4

### Determination of enzymatic activity of neprilysin in vitro (apparent Km)

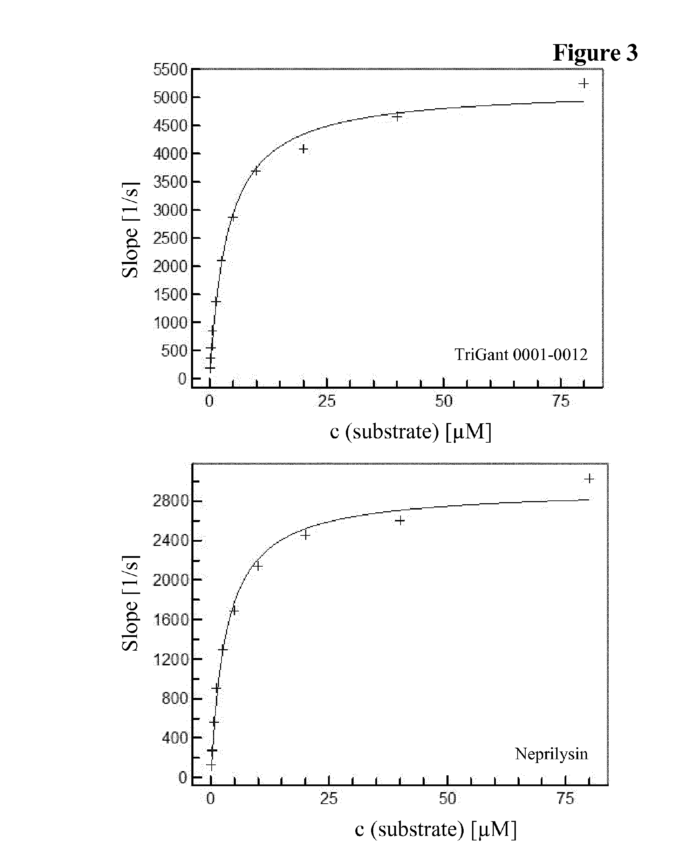

The 20 µl assay was performed on low-volume black Costar 384-well plates at 25°C. A working solution of 160 µM peptide substrate MCA-RPPGFSAFK(Dnp)-OH (R&D Systems Cat. No. ES005) was prepared in 50 mM Tris-HCl pH7.8, 25 mM NaCl and 5 mM ZnCl₂. 10 µl of Neprilysin (R&D Systems, Cat. No 1182-ZNC) or Neprilysin fusion polypeptide, diluted to 1 nM in assay buffer, were transferred to plate. For determination of apparent Kₘ values various concentrations of substrate (0.078-80 nM in 2-fold dilutions dilutions) were added and the enzyme reaction started. The fluorescence increase was monitored with excitation at 320 nm and emission at 405 nm on an Envision Reader. Hydrolysis rates and apparent Km values were calculated using XLfit® software (IDBS) (see Figure 3).

| | Vₘₐₓ [1/s] | Km [µM] |
|---|---|---|
| TriGant 0001-0012 | 5169 | 3.8 |
| neprilysin (RnD Systems) | 2926 | 3.2 |

### Example 5

### Staining of fusion polypeptide to native human β-amyloid plaques from brain sections of an Alzheimer's disease patient by indirect immunofluorescence

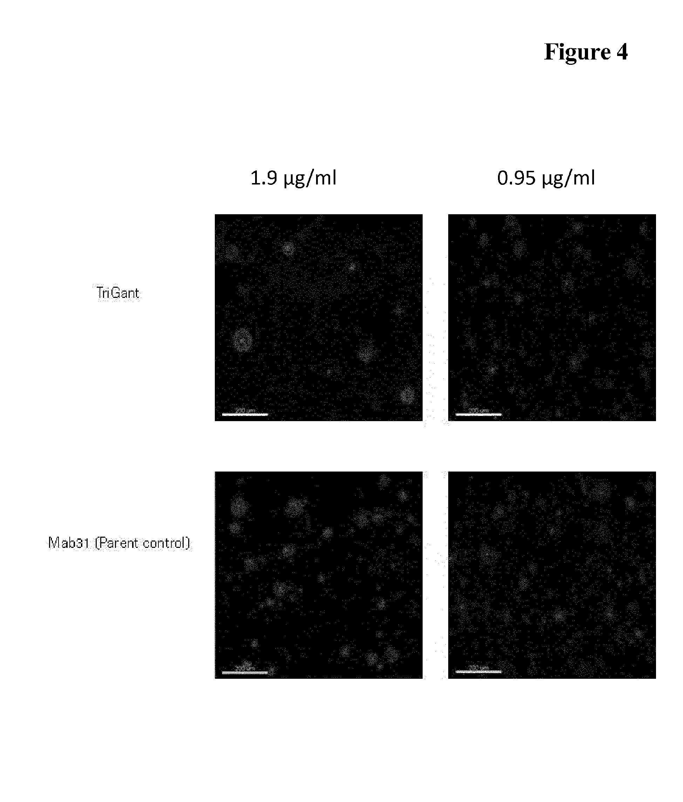

Fusion polypeptide was tested for the ability to stain native human β-amyloid plaques by immunohistochemistry analysis using indirect immunofluorescence. Specific and sensitive staining of genuine human β-amyloid plaques was demonstrated. Cryostat sections of unfixed tissue from the temporal cortex obtained postmortem from patients positively diagnosed for Alzheimer's disease were labeled by indirect immunofluorescence. A two-step incubation was used to detect bound fusion polypeptide, which was revealed by affinity-purified goat anti-human (GAH555) IgG (H+L) conjugated to Alexa 555 dye (Molecular Probes). Controls included unrelated human IgG1 antibodies (Sigma) and the secondary antibody alone, which all gave negative results. All types of β-amyloid plaques were clearly and consistently revealed at fusion polypeptide concentrations tested from 10 ng/ml to 5 µg/ml Specific and sensitive staining of genuine human amyloid-β plaques is shown for fusion polypeptide at a concentration of 1 µg/ml (see Figure 4).

### Example 6

### In vivo β-amyloid plaque decoration by fusion polypeptide in a mouse model of Alzheimer's disease

Fusion polypeptide was tested in APP/PS2 double transgenic mice, a mouse model for AD-related amyloidosis (Richards, J. Neuroscience 23 (2003) 8989-9003) for their ability to immuno-decorate β-amyloid plaques in vivo. This enabled assessment of the extent of brain penetration and binding to amyloid-β plaques. The fusion polypeptide was administered at different doses compared to naked anti-Aβ monoclonal antibody and after 6 days animals were perfused with phosphate-buffered saline and the brains frozen on dry ice and prepared for cryosectioning. The fusion polypeptide showed substantially improved and highly effective brain penetration in vivo (as compared to the naked anti-Aβ monoclonal antibody).

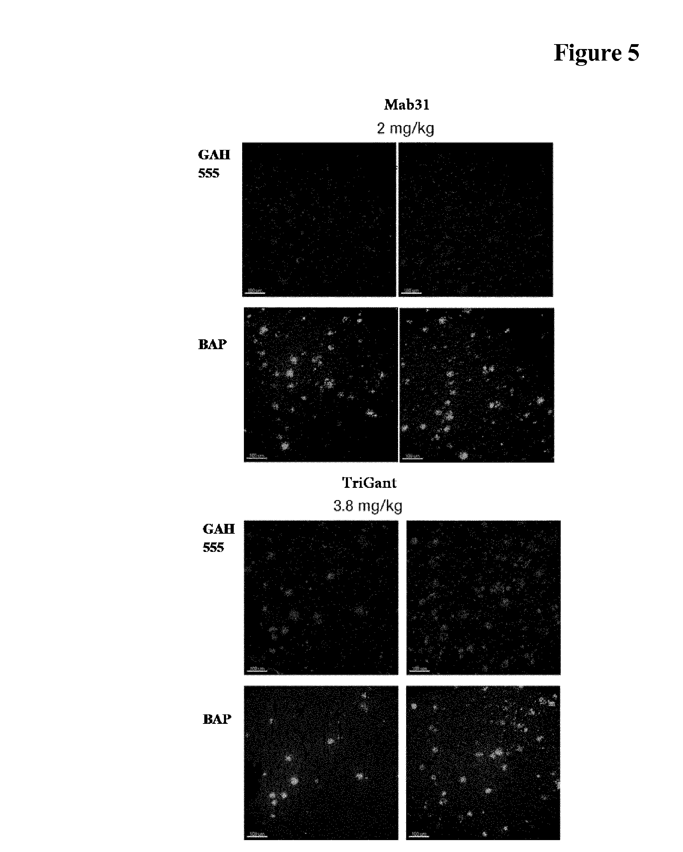

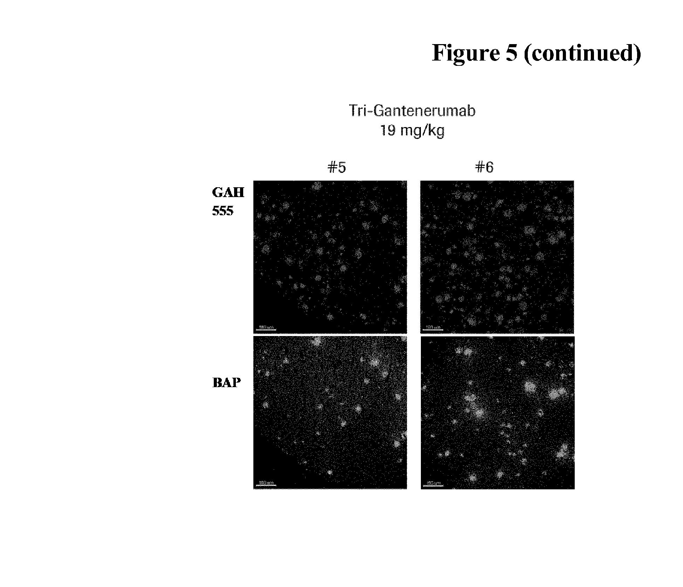

The presence of the antibodies bound to β-amyloid plaques was assessed using unfixed cryostat sections either by single-labeled indirect immunofluorescence with goat anti-human IgG (H+L) conjugated to Alexa555 dye (GAH555) (Molecular Probes) at a concentration of 15 µg /ml for 1 hour at room temperature. A counterstaining for amyloid plaques was done by incubation with BAP-2, a mouse monoclonal antibody against Aβ conjugated to Alexa 488 at a concentration of 0.5 µg /ml for 1 hour at room temperature. Slides were embedded with fluorescence mounting medium (S3023 Dako) and imaging was done by confocal laser microscopy (Figure 5).

At equimolar doses (2 and 3.8 mg/kg) fusion polypeptide were found to cross substantially better the blood brain barrier and strongly immuno-decorate all β-amyloid plaques in vivo. Representative images shown in Figure 5 demonstrate the improved binding capacity of the fusion polypeptide compared to the naked monoclonal antibody which crosses the blood-brain border at much lower extent.

Efficacy to lower plaque load in huAPP/PS2 double transgenic mice will be established in a chronic multiple dose study with weekly IV injections. Comparative analysis of fusion polypeptide and naked anti-Aβ mAb without modifications are expected to reveal a significantly higher capacity to lower plaque load.

## Claims

1. A method for the recombinant production of a polypeptide in a mammalian cell comprising the following steps:
- cultivating a mammalian cell expressing the polypeptide as fusion polypeptide with human neprilysin whereby the neprilysin is fused to the C-terminus of the polypeptide, and
- recovering the polypeptide from the cell or the cultivation medium and thereby producing the polypeptide.

2. Use of human neprilysin fused to the C-terminus of a polypeptide for increasing the expression yield of the polypeptide in a mammalian cell.

3. The method or use according to any one of claims 1 to 2, **characterized in that** the neprilysin is a neprilysin fragment consisting of amino acid residues 52 to 750 of SEQ ID NO: 04.

4. The method or use according to any one of claims 1 to 3, **characterized in that** the neprilysin is fused via a linker polypeptide to the C-terminus of the polypeptide.

5. The method or use according to claim 4, **characterized in that** the linker is a glycine-serine-linker.

6. The method or use according to any one of claims 1 to 5, **characterized in that** the fusion polypeptide comprises an enzymatic cleavage site between the polypeptide and the neprilysin or the linker.

7. The method or use according to any one of claims 1 to 6, **characterized in that** the mammalian cell is a HEK cell or a CHO cell.

8. The method according to any one of claims 1 to 7, **characterized in that** the method comprises the following steps:
- cultivating a mammalian cell expressing the polypeptide as fusion polypeptide with human neprilysin whereby the neprilysin is fused to the C-terminus of the polypeptide,
- recovering the polypeptide from the cell or the cultivation medium, and
- enzymatically cleaving the fusion polypeptide and thereby producing the polypeptide.

9. The method or use according to any one of claims 1 to 8, **characterized in that** the polypeptide is an antibody.

10. The method or use according to claim 9, **characterized in that** the neprilysin is fused to the C-terminus of one heavy chain of the antibody.


SEQUENCE LISTING

<110>  F. Hoffmann-La Roche AG

<120>   Method for improving the recombinant expression of a polypeptide
        by C-terminal fusion to human neprilysin

<130>  31704 EP

<160>  22

<170>  PatentIn version 3.5

<210>  1
<211>  454
<212>  PRT
<213>  Artificial Sequence

<220>
<223>  mAb31-IgG1 heavy chain without C-terminal GK

<400>  1

Gln Val Glu Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Asn Ala Ser Gly Thr Arg Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Lys Gly Asn Thr His Lys Pro Tyr Gly Tyr Val Arg Tyr
            100                 105                 110

Phe Asp Val Trp Gly Gln Gly Thr Leu Val Thr Val Ser Ser Ala Ser
        115                 120                 125

Thr Lys Gly Pro Ser Val Phe Pro Leu Ala Pro Ser Ser Lys Ser Thr
    130                 135                 140

Ser Gly Gly Thr Ala Ala Leu Gly Cys Leu Val Lys Asp Tyr Phe Pro
145                 150                 155                 160

Glu Pro Val Thr Val Ser Trp Asn Ser Gly Ala Leu Thr Ser Gly Val
                165                 170                 175

His Thr Phe Pro Ala Val Leu Gln Ser Ser Gly Leu Tyr Ser Leu Ser
            180                 185                 190

Ser Val Val Thr Val Pro Ser Ser Ser Leu Gly Thr Gln Thr Tyr Ile
        195                 200                 205

Cys Asn Val Asn His Lys Pro Ser Asn Thr Lys Val Asp Lys Lys Val
    210                 215                 220

Glu Pro Lys Ser Cys Asp Lys Thr His Thr Cys Pro Pro Cys Pro Ala
225                 230                 235                 240

Pro Glu Leu Leu Gly Gly Pro Ser Val Phe Leu Phe Pro Pro Lys Pro
                245                 250                 255

Lys Asp Thr Leu Met Ile Ser Arg Thr Pro Glu Val Thr Cys Val Val
            260                 265                 270

Val Asp Val Ser His Glu Asp Pro Glu Val Lys Phe Asn Trp Tyr Val
        275                 280                 285

Asp Gly Val Glu Val His Asn Ala Lys Thr Lys Pro Arg Glu Glu Gln
    290                 295                 300

Tyr Asn Ser Thr Tyr Arg Val Val Ser Val Leu Thr Val Leu His Gln
305                 310                 315                 320

Asp Trp Leu Asn Gly Lys Glu Tyr Lys Cys Lys Val Ser Asn Lys Ala
                325                 330                 335

Leu Pro Ala Pro Ile Glu Lys Thr Ile Ser Lys Ala Lys Gly Gln Pro
            340                 345                 350

Arg Glu Pro Gln Val Tyr Thr Leu Pro Pro Ser Arg Asp Glu Leu Thr
        355                 360                 365

Lys Asn Gln Val Ser Leu Thr Cys Leu Val Lys Gly Phe Tyr Pro Ser
    370                 375                 380

Asp Ile Ala Val Glu Trp Glu Ser Asn Gly Gln Pro Glu Asn Asn Tyr
385                 390                 395                 400

Lys Thr Thr Pro Pro Val Leu Asp Ser Asp Gly Ser Phe Phe Leu Tyr
                405                 410                 415

Ser Lys Leu Thr Val Asp Lys Ser Arg Trp Gln Gln Gly Asn Val Phe
            420                 425                 430

Ser Cys Ser Val Met His Glu Ala Leu His Asn His Tyr Thr Gln Lys
        435                 440                 445

Ser Leu Ser Leu Ser Pro
    450

<210> 2
<211> 454
<212> PRT
<213> Artificial Sequence

<220>
<223> mAb31-IgG1-HC knob without C-terminal GK

<400> 2

Gln Val Glu Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Asn Ala Ser Gly Thr Arg Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Lys Gly Asn Thr His Lys Pro Tyr Gly Tyr Val Arg Tyr
            100                 105                 110

Phe Asp Val Trp Gly Gln Gly Thr Leu Val Thr Val Ser Ser Ala Ser
        115                 120                 125

Thr Lys Gly Pro Ser Val Phe Pro Leu Ala Pro Ser Ser Lys Ser Thr
    130                 135                 140

Ser Gly Gly Thr Ala Ala Leu Gly Cys Leu Val Lys Asp Tyr Phe Pro
145                 150                 155                 160

Glu Pro Val Thr Val Ser Trp Asn Ser Gly Ala Leu Thr Ser Gly Val
                165                 170                 175

His Thr Phe Pro Ala Val Leu Gln Ser Ser Gly Leu Tyr Ser Leu Ser
            180                 185                 190

Ser Val Val Thr Val Pro Ser Ser Ser Leu Gly Thr Gln Thr Tyr Ile
        195                 200                 205

Cys Asn Val Asn His Lys Pro Ser Asn Thr Lys Val Asp Lys Lys Val
    210                 215                 220

Glu Pro Lys Ser Cys Asp Lys Thr His Thr Cys Pro Pro Cys Pro Ala
225                 230                 235                 240

Pro Glu Leu Leu Gly Gly Pro Ser Val Phe Leu Phe Pro Pro Lys Pro
                245                 250                 255

Lys Asp Thr Leu Met Ile Ser Arg Thr Pro Glu Val Thr Cys Val Val
            260                 265                 270

Val Asp Val Ser His Glu Asp Pro Glu Val Lys Phe Asn Trp Tyr Val
        275                 280                 285

Asp Gly Val Glu Val His Asn Ala Lys Thr Lys Pro Arg Glu Glu Gln
    290                 295                 300

Tyr Asn Ser Thr Tyr Arg Val Val Ser Val Leu Thr Val Leu His Gln
305                 310                 315                 320

Asp Trp Leu Asn Gly Lys Glu Tyr Lys Cys Lys Val Ser Asn Lys Ala
                325                 330                 335

Leu Pro Ala Pro Ile Glu Lys Thr Ile Ser Lys Ala Lys Gly Gln Pro
            340                 345                 350

Arg Glu Pro Gln Val Tyr Thr Leu Pro Pro Cys Arg Asp Glu Leu Thr
        355                 360                 365

Lys Asn Gln Val Ser Leu Trp Cys Leu Val Lys Gly Phe Tyr Pro Ser
    370                 375                 380

Asp Ile Ala Val Glu Trp Glu Ser Asn Gly Gln Pro Glu Asn Asn Tyr
385                 390                 395                 400

Lys Thr Thr Pro Pro Val Leu Asp Ser Asp Gly Ser Phe Phe Leu Tyr
                405                 410                 415

Ser Lys Leu Thr Val Asp Lys Ser Arg Trp Gln Gln Gly Asn Val Phe
            420                 425                 430

Ser Cys Ser Val Met His Glu Ala Leu His Asn His Tyr Thr Gln Lys
        435                 440                 445

Ser Leu Ser Leu Ser Pro
    450

<210> 3
<211> 1172
<212> PRT
<213> Artificial Sequence

<220>
<223> mAb31-IgG1-neprilysin

<400> 3

<210> 4
<211> 750
<212> PRT
<213> Homo sapiens

<400> 4

<210>  5
<211>  940
<212>  PRT
<213>  Artificial Sequence

<220>
<223>  MAB31-IGG1-KNOB-SS_G4S-4_VL-8D3-CK_G4S-6-GG_VH-8D3-CH1

<400>  5

Gln Val Glu Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Asn Ala Ser Gly Thr Arg Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Lys Gly Asn Thr His Lys Pro Tyr Gly Tyr Val Arg Tyr
            100                 105                 110

Phe Asp Val Trp Gly Gln Gly Thr Leu Val Thr Val Ser Ser Ala Ser
        115                 120                 125

Thr Lys Gly Pro Ser Val Phe Pro Leu Ala Pro Ser Ser Lys Ser Thr
    130                 135                 140

Ser Gly Gly Thr Ala Ala Leu Gly Cys Leu Val Lys Asp Tyr Phe Pro
145                 150                 155                 160

Glu Pro Val Thr Val Ser Trp Asn Ser Gly Ala Leu Thr Ser Gly Val
                165                 170                 175

His Thr Phe Pro Ala Val Leu Gln Ser Ser Gly Leu Tyr Ser Leu Ser
        180                 185                 190

Ser Val Val Thr Val Pro Ser Ser Ser Leu Gly Thr Gln Thr Tyr Ile
    195                 200                 205

Cys Asn Val Asn His Lys Pro Ser Asn Thr Lys Val Asp Lys Lys Val
    210                 215                 220

Glu Pro Lys Ser Cys Asp Lys Thr His Thr Cys Pro Pro Cys Pro Ala
225                 230                 235                 240

Pro Glu Leu Leu Gly Gly Pro Ser Val Phe Leu Phe Pro Pro Lys Pro
                245                 250                 255

Lys Asp Thr Leu Met Ile Ser Arg Thr Pro Glu Val Thr Cys Val Val
        260                 265                 270

Val Asp Val Ser His Glu Asp Pro Glu Val Lys Phe Asn Trp Tyr Val
    275                 280                 285

Asp Gly Val Glu Val His Asn Ala Lys Thr Lys Pro Arg Glu Glu Gln
    290                 295                 300

Tyr Asn Ser Thr Tyr Arg Val Val Ser Val Leu Thr Val Leu His Gln
305                 310                 315                 320

Asp Trp Leu Asn Gly Lys Glu Tyr Lys Cys Lys Val Ser Asn Lys Ala
                325                 330                 335

Leu Pro Ala Pro Ile Glu Lys Thr Ile Ser Lys Ala Lys Gly Gln Pro
        340                 345                 350

Arg Glu Pro Gln Val Tyr Thr Leu Pro Pro Cys Arg Asp Glu Leu Thr
    355                 360                 365

Lys Asn Gln Val Ser Leu Trp Cys Leu Val Lys Gly Phe Tyr Pro Ser
    370                 375                 380

Asp Ile Ala Val Glu Trp Glu Ser Asn Gly Gln Pro Glu Asn Asn Tyr
385                 390                 395                 400

Lys Thr Thr Pro Pro Val Leu Asp Ser Asp Gly Ser Phe Phe Leu Tyr
                405                 410                 415

Ser Lys Leu Thr Val Asp Lys Ser Arg Trp Gln Gln Gly Asn Val Phe
        420                 425                 430

Ser Cys Ser Val Met His Glu Ala Leu His Asn His Tyr Thr Gln Lys
        435                 440                 445

Ser Leu Ser Leu Ser Pro Gly Gly Gly Ser Gly Gly Gly Gly Ser Gly
    450                 455                 460

Gly Gly Gly Ser Gly Gly Gly Gly Ser Asp Ile Gln Met Thr Gln Ser
465                 470                 475                 480

Pro Ala Ser Leu Ser Ala Ser Leu Glu Glu Ile Val Thr Ile Thr Cys
                485                 490                 495

Gln Ala Ser Gln Asp Ile Gly Asn Trp Leu Ala Trp Tyr Gln Gln Lys
            500                 505                 510

Pro Gly Lys Ser Pro Gln Leu Leu Ile Tyr Gly Ala Thr Ser Leu Ala
        515                 520                 525

Asp Gly Val Pro Ser Arg Phe Ser Gly Ser Arg Ser Gly Thr Gln Phe
    530                 535                 540

Ser Leu Lys Ile Ser Arg Val Gln Val Glu Asp Ile Gly Ile Tyr Tyr
545                 550                 555                 560

Cys Leu Gln Ala Tyr Asn Thr Pro Trp Thr Phe Gly Gly Gly Thr Lys
                565                 570                 575

Val Glu Ile Lys Arg Thr Val Ala Ala Pro Ser Val Phe Ile Phe Pro
            580                 585                 590

Pro Ser Asp Glu Gln Leu Lys Ser Gly Thr Ala Ser Val Val Cys Leu
        595                 600                 605

Leu Asn Asn Phe Tyr Pro Arg Glu Ala Lys Val Gln Trp Lys Val Asp
    610                 615                 620

Asn Ala Leu Gln Ser Gly Asn Ser Gln Glu Ser Val Thr Glu Gln Asp
625                 630                 635                 640

Ser Lys Asp Ser Thr Tyr Ser Leu Ser Ser Thr Leu Thr Leu Ser Lys
                645                 650                 655

Ala Asp Tyr Glu Lys His Lys Val Tyr Ala Cys Glu Val Thr His Gln
            660                 665                 670

Gly Leu Ser Ser Pro Val Thr Lys Ser Phe Asn Arg Gly Glu Cys Gly

        675                 680                 685

Gly Gly Gly Ser Gly Gly Gly Gly Ser Gly Gly Gly Gly Ser Gly Gly
    690                 695                 700

Gly Gly Ser Gly Gly Gly Gly Ser Gly Gly Gly Gly Ser Gly Gly Glu
705                 710                 715                 720

Val Gln Leu Val Glu Ser Gly Gly Gly Leu Val Gln Pro Gly Asn Ser
                725                 730                 735

Leu Thr Leu Ser Cys Val Ala Ser Gly Phe Thr Phe Ser Asn Tyr Gly
            740                 745                 750

Met His Trp Ile Arg Gln Ala Pro Lys Lys Gly Leu Glu Trp Ile Ala
        755                 760                 765

Met Ile Tyr Tyr Asp Ser Ser Lys Met Asn Tyr Ala Asp Thr Val Lys
    770                 775                 780

Gly Arg Phe Thr Ile Ser Arg Asp Asn Ser Lys Asn Thr Leu Tyr Leu
785                 790                 795                 800

Glu Met Asn Ser Leu Arg Ser Glu Asp Thr Ala Met Tyr Tyr Cys Ala
                805                 810                 815

Val Pro Thr Ser His Tyr Val Val Asp Val Trp Gly Gln Gly Val Ser
            820                 825                 830

Val Thr Val Ser Ser Ala Ser Thr Lys Gly Pro Ser Val Phe Pro Leu
        835                 840                 845

Ala Pro Ser Ser Lys Ser Thr Ser Gly Gly Thr Ala Ala Leu Gly Cys
    850                 855                 860

Leu Val Lys Asp Tyr Phe Pro Glu Pro Val Thr Val Ser Trp Asn Ser
865                 870                 875                 880

Gly Ala Leu Thr Ser Gly Val His Thr Phe Pro Ala Val Leu Gln Ser
                885                 890                 895

Ser Gly Leu Tyr Ser Leu Ser Ser Val Val Thr Val Pro Ser Ser Ser
            900                 905                 910

Leu Gly Thr Gln Thr Tyr Ile Cys Asn Val Asn His Lys Pro Ser Asn
        915                 920                 925

Thr Lys Val Asp Lys Lys Val Glu Pro Lys Ser Cys
    930                 935                 940

<210>  6
<211>  1168
<212>  PRT
<213>  Artificial Sequence

<220>
<223>  MAB31_8D3_HC-HOLE_NEPRI

<400>  6

Gln Val Glu Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Asn Ala Ser Gly Thr Arg Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Lys Gly Asn Thr His Lys Pro Tyr Gly Tyr Val Arg Tyr
            100                 105                 110

Phe Asp Val Trp Gly Gln Gly Thr Leu Val Thr Val Ser Ser Ala Ser
        115                 120                 125

Thr Lys Gly Pro Ser Val Phe Pro Leu Ala Pro Ser Ser Lys Ser Thr
    130                 135                 140

Ser Gly Gly Thr Ala Ala Leu Gly Cys Leu Val Lys Asp Tyr Phe Pro
145                 150                 155                 160

Glu Pro Val Thr Val Ser Trp Asn Ser Gly Ala Leu Thr Ser Gly Val
                165                 170                 175

His Thr Phe Pro Ala Val Leu Gln Ser Ser Gly Leu Tyr Ser Leu Ser
            180                 185                 190

Ser Val Val Thr Val Pro Ser Ser Ser Leu Gly Thr Gln Thr Tyr Ile

195                 200                 205

Cys Asn Val Asn His Lys Pro Ser Asn Thr Lys Val Asp Lys Lys Val
    210                 215                 220

Glu Pro Lys Ser Cys Asp Lys Thr His Thr Cys Pro Pro Cys Pro Ala
225                 230                 235                 240

Pro Glu Leu Leu Gly Gly Pro Ser Val Phe Leu Phe Pro Pro Lys Pro
                245                 250                 255

Lys Asp Thr Leu Met Ile Ser Arg Thr Pro Glu Val Thr Cys Val Val
            260                 265                 270

Val Asp Val Ser His Glu Asp Pro Glu Val Lys Phe Asn Trp Tyr Val
        275                 280                 285

Asp Gly Val Glu Val His Asn Ala Lys Thr Lys Pro Arg Glu Glu Gln
    290                 295                 300

Tyr Asn Ser Thr Tyr Arg Val Val Ser Val Leu Thr Val Leu His Gln
305                 310                 315                 320

Asp Trp Leu Asn Gly Lys Glu Tyr Lys Cys Lys Val Ser Asn Lys Ala
                325                 330                 335

Leu Pro Ala Pro Ile Glu Lys Thr Ile Ser Lys Ala Lys Gly Gln Pro
            340                 345                 350

Arg Glu Pro Gln Val Cys Thr Leu Pro Pro Ser Arg Asp Glu Leu Thr
        355                 360                 365

Lys Asn Gln Val Ser Leu Ser Cys Ala Val Lys Gly Phe Tyr Pro Ser
    370                 375                 380

Asp Ile Ala Val Glu Trp Glu Ser Asn Gly Gln Pro Glu Asn Asn Tyr
385                 390                 395                 400

Lys Thr Thr Pro Pro Val Leu Asp Ser Asp Gly Ser Phe Phe Leu Val
                405                 410                 415

Ser Lys Leu Thr Val Asp Lys Ser Arg Trp Gln Gln Gly Asn Val Phe
            420                 425                 430

Ser Cys Ser Val Met His Glu Ala Leu His Asn His Tyr Thr Gln Lys
        435                 440                 445

Ser Leu Ser Leu Ser Pro Gly Gly Gly Gly Ser Gly Gly Gly Gly Ser
    450                 455                 460

Gly Gly Gly Gly Ser Tyr Asp Asp Gly Ile Cys Lys Ser Ser Asp Cys
465                 470                 475                 480

Ile Lys Ser Ala Ala Arg Leu Ile Gln Asn Met Asp Ala Thr Thr Glu
                485                 490                 495

Pro Cys Thr Asp Phe Phe Lys Tyr Ala Cys Gly Gly Trp Leu Lys Arg
            500                 505                 510

Asn Val Ile Pro Glu Thr Ser Ser Arg Tyr Gly Asn Phe Asp Ile Leu
        515                 520                 525

Arg Asp Glu Leu Glu Val Val Leu Lys Asp Val Leu Gln Glu Pro Lys
    530                 535                 540

Thr Glu Asp Ile Val Ala Val Gln Lys Ala Lys Ala Leu Tyr Arg Ser
545                 550                 555                 560

Cys Ile Asn Glu Ser Ala Ile Asp Ser Arg Gly Gly Glu Pro Leu Leu
                565                 570                 575

Lys Leu Leu Pro Asp Ile Tyr Gly Trp Pro Val Ala Thr Glu Asn Trp
            580                 585                 590

Glu Gln Lys Tyr Gly Ala Ser Trp Thr Ala Glu Lys Ala Ile Ala Gln
        595                 600                 605

Leu Asn Ser Lys Tyr Gly Lys Lys Val Leu Ile Asn Leu Phe Val Gly
    610                 615                 620

Thr Asp Asp Lys Asn Ser Val Asn His Val Ile His Ile Asp Gln Pro
625                 630                 635                 640

Arg Leu Gly Leu Pro Ser Arg Asp Tyr Tyr Glu Cys Thr Gly Ile Tyr
                645                 650                 655

Lys Glu Ala Cys Thr Ala Tyr Val Asp Phe Met Ile Ser Val Ala Arg
            660                 665                 670

Leu Ile Arg Gln Glu Glu Arg Leu Pro Ile Asp Glu Asn Gln Leu Ala
        675                 680                 685

Leu Glu Met Asn Lys Val Met Glu Leu Glu Lys Glu Ile Ala Asn Ala
    690                 695                 700

Thr Ala Lys Pro Glu Asp Arg Asn Asp Pro Met Leu Leu Tyr Asn Lys
705                 710                 715                 720

Met Thr Leu Ala Gln Ile Gln Asn Asn Phe Ser Leu Glu Ile Asn Gly
                725                 730                 735

Lys Pro Phe Ser Trp Leu Asn Phe Thr Asn Glu Ile Met Ser Thr Val
            740                 745                 750

Asn Ile Ser Ile Thr Asn Glu Glu Asp Val Val Val Tyr Ala Pro Glu
        755                 760                 765

Tyr Leu Thr Lys Leu Lys Pro Ile Leu Thr Lys Tyr Ser Ala Arg Asp
    770                 775                 780

Leu Gln Asn Leu Met Ser Trp Arg Phe Ile Met Asp Leu Val Ser Ser
785                 790                 795                 800

Leu Ser Arg Thr Tyr Lys Glu Ser Arg Asn Ala Phe Arg Lys Ala Leu
                805                 810                 815

Tyr Gly Thr Thr Ser Glu Thr Ala Thr Trp Arg Arg Cys Ala Asn Tyr
            820                 825                 830

Val Asn Gly Asn Met Glu Asn Ala Val Gly Arg Leu Tyr Val Glu Ala
        835                 840                 845

Ala Phe Ala Gly Glu Ser Lys His Val Val Glu Asp Leu Ile Ala Gln
    850                 855                 860

Ile Arg Glu Val Phe Ile Gln Thr Leu Asp Asp Leu Thr Trp Met Asp
865                 870                 875                 880

Ala Glu Thr Lys Lys Arg Ala Glu Glu Lys Ala Leu Ala Ile Lys Glu
                885                 890                 895

Arg Ile Gly Tyr Pro Asp Asp Ile Val Ser Asn Asp Asn Lys Leu Asn
            900                 905                 910

Asn Glu Tyr Leu Glu Leu Asn Tyr Lys Glu Asp Glu Tyr Phe Glu Asn
        915                 920                 925

Ile Ile Gln Asn Leu Lys Phe Ser Gln Ser Lys Gln Leu Lys Lys Leu
    930                 935                 940

Arg Glu Lys Val Asp Lys Asp Glu Trp Ile Ser Gly Ala Ala Val Val
945                 950                 955                 960

Asn Ala Phe Tyr Ser Ser Gly Arg Asn Gln Ile Val Phe Pro Ala Gly
                965                 970                 975

Ile Leu Gln Pro Pro Phe Phe Ser Ala Gln Gln Ser Asn Ser Leu Asn
            980                 985                 990

Tyr Gly Gly Ile Gly Met Val Ile Gly His Glu Ile Thr His Gly Phe
        995                 1000                1005

Asp Asp Asn Gly Arg Asn Phe Asn Lys Asp Gly Asp Leu Val Asp
    1010                1015                1020

Trp Trp Thr Gln Gln Ser Ala Ser Asn Phe Lys Glu Gln Ser Gln
    1025                1030                1035

Cys Met Val Tyr Gln Tyr Gly Asn Phe Ser Trp Asp Leu Ala Gly
    1040                1045                1050

Gly Gln His Leu Asn Gly Ile Asn Thr Leu Gly Glu Asn Ile Ala
    1055                1060                1065

Asp Asn Gly Gly Leu Gly Gln Ala Tyr Arg Ala Tyr Gln Asn Tyr
    1070                1075                1080

Ile Lys Lys Asn Gly Glu Glu Lys Leu Leu Pro Gly Leu Asp Leu
    1085                1090                1095

Asn His Lys Gln Leu Phe Phe Leu Asn Phe Ala Gln Val Trp Cys
    1100                1105                1110

Gly Thr Tyr Arg Pro Glu Tyr Ala Val Asn Ser Ile Lys Thr Asp
    1115                1120                1125

Val His Ser Pro Gly Asn Phe Arg Ile Ile Gly Thr Leu Gln Asn
    1130                1135                1140

Ser Ala Glu Phe Ser Glu Ala Phe His Cys Arg Lys Asn Ser Tyr
    1145                1150                1155

Met Asn Pro Glu Lys Lys Cys Arg Val Trp
    1160                1165

<210> 7
<211> 215
<212> PRT
<213> Artificial Sequence

<220>
<223>  TPIP:5170_MAB31-LC

<400>  7

Asp Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Val Pro Ala Arg Phe Ser
    50                  55                  60

Gly Ser Gly Ser Gly Thr Asp Phe Thr Leu Thr Ile Ser Ser Leu Glu
65                  70                  75                  80

Pro Glu Asp Phe Ala Thr Tyr Tyr Cys Leu Gln Ile Tyr Asn Met Pro
                85                  90                  95

Ile Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  8
<211>  5

<212>  PRT
<213>  Artificial Sequence

<220>
<223>  protease cleavage site

<400>  8

Pro Ala Pro Ser Pro
1               5


<210>  9
<211>  4
<212>  PRT
<213>  Artificial Sequence

<220>
<223>  protease cleavage site

<400>  9

Pro Pro Ser Pro
1


<210>  10
<211>  4
<212>  PRT
<213>  Artificial Sequence

<220>
<223>  protease cleavage site

<400>  10

Pro Pro Ala Pro
1


<210>  11
<211>  4
<212>  PRT
<213>  Artificial Sequence

<220>
<223>  protease cleavage site

<400>  11

Pro Pro Thr Pro
1


<210>  12
<211>  4
<212>  PRT
<213>  Artificial Sequence

<220>
<223>  protease cleavage site

<400>  12

Pro Pro Gly Pro
1

<210> 13
<211> 6
<212> PRT
<213> Artificial Sequence

<220>
<223> enzymatic cleavage site

<400> 13

Pro Arg Pro Pro Thr Pro
1               5

<210> 14
<211> 7
<212> PRT
<213> Artificial Sequence

<220>
<223> enzymatic cleavage site

<400> 14

Val Val Ala Pro Pro Ala Pro
1               5

<210> 15
<211> 7
<212> PRT
<213> Artificial Sequence

<220>
<223> enzymatic cleavage site

<400> 15

Val Val Ala Pro Pro Ser Pro
1               5

<210> 16
<211> 7
<212> PRT
<213> Artificial Sequence

<220>
<223> enzymatic cleavage site

<400> 16

Val Val Ala Pro Pro Thr Pro
1               5

<210> 17
<211> 7

<212>  PRT
<213>  Artificial Sequence

<220>
<223>  enzymatic cleavage site

<400>  17

Val Val Ala Pro Pro Gly Pro
1               5


<210>  18
<211>  6
<212>  PRT
<213>  Artificial Sequence

<220>
<223>  enzymatic cleavage site

<400>  18

Pro Arg Pro Pro Thr Pro
1               5


<210>  19
<211>  6
<212>  PRT
<213>  Artificial Sequence

<220>
<223>  enzymatic cleavage site

<400>  19

Ala Pro Pro Ala Ala Pro
1               5


<210>  20
<211>  6
<212>  PRT
<213>  Artificial Sequence

<220>
<223>  enzymatic cleavage site

<400>  20

Pro Arg Pro Pro Ala Pro
1               5


<210>  21
<211>  6
<212>  PRT
<213>  Artificial Sequence

<220>
<223>  enzymatic cleavage site

<400>  21

Pro Arg Pro Pro Ser Pro
1               5

<210>  22
<211>  6
<212>  PRT
<213>  Artificial Sequence

<220>
<223>  enzymatic cleavage site

<400>  22

Pro Arg Pro Pro Gly Pro
1               5